# EUROPEAN PATENT SPECIFICATION

(11) **EP 3 701 851 B1**
(45) Date of publication and mention of the grant of the patent: **17.09.2025**
(21) Application number: 20159693.9
(22) Date of filing: 27.02.2020
(51) Int. Cl.: A61B 1/00, A61B 1/018

(54) **ENDOSCOPE**
ENDOSKOP
ENDOSCOPE

(30) Priority: 01.03.2019 JP 2019037790
(43) Date of publication of application: 02.09.2020
(73) Proprietor: FUJIFILM Corporation, Tokyo 106-8620 (JP)
(72) Inventor: HARADA, Takashi, Ashigarakami-gun, Kanagawa, 258-8538 (JP)
(74) Representative: Klunker IP Patentanwälte PartG mbB

(56) References cited:
- EP-A1- 1 764 028
- JP-A- 2014 046 167
- US-A1- 2017 000 317

## Description

### BACKGROUND OF THE INVENTION

### 1. Field of the Invention

The present invention relates to an endoscope, and more particularly, to an endoscope comprising an elevator that is provided on the distal end side of an insertion unit and changes the lead-out direction of a treatment tool.

### 2. Description of the Related Art

In an endoscope, various treatment tools are introduced from a treatment tool inlet provided in an operation unit, and the treatment tools are led out of the treatment tool outlet opened to the distal end part of the insertion unit to be used for treatment. For example, a treatment tool, such as a guide wire or a contrast tube, is used in a duodenoscope. A treatment tool, such as a puncture needle, is used in an ultrasound endoscope. A treatment tool, such as forceps or a snare, is used in other direct-viewing endoscopes and oblique-viewing endoscopes. The lead-out direction of such a treatment tool needs to be changed at a distal end part thereof to perform treatment at a desired position in an object to be examined. For this purpose, the distal end part is provided with an elevator that changes the lead-out direction of the treatment tool. Further, the endoscope is provided with a treatment tool-standing mechanism that changes the attitude of the elevator between a standing position and a fallen position.

JP4970877B discloses the structure of an endoscope comprising an elevator. JP4970877B discloses an endoscope comprising a hard distal-end-part body that is provided with a contact member made of an insulating material. The treatment tool is pressed and fixed by the elevator and the contact member as the treatment tool is operated to stand by the elevator.

US2017000317A1 discloses a distal end structure of endoscope that includes a distal end member formed of a metal material, an insulating ring provided in an annular shape around an insertion axis on a proximal end side of the distal end member, a cover formed of a flexible insulative material that covers the distal end member, a projecting section provided integrally with the insulating ring and projecting in a direction crossing the insertion axis, a through-hole provided in the cover and engaging with the projecting section, and a to-be-tom-apart section provided near the through-hole, in which force is applied to the to-be-torn-apart section to tear apart the cover along the insertion axis direction and remove the cover from the distal end member.

### SUMMARY OF THE INVENTION

Incidentally, the contact member receives a force through the treatment tool as the elevator is operated to stand. In a case where the contact member receives a force in JP4970877B, the contact member is deformed to be turned in the standing direction of the elevator and is moved from the distal-end-part body. For this reason, there is a concern that a positional deviation occurs between the contact member and the distal-end-part body.

The invention has been made in consideration of the above-mentioned circumstances, and an object of the invention is to provide an endoscope that can prevent the deformation of a contact member and suppress a positional deviation.

In a first aspect of the invention there is provided an endoscope according to claim 1 of the appended claims.

According to a second aspect of the invention, the restriction surface is formed of an inclined surface that faces the rotating shaft toward the elevator. The second aspect prescribes one preferred form of the stopper portion.

According to a third aspect of the invention, the restriction surface is formed of a parallel surface that extends toward the elevator and is parallel to a direction of a longitudinal axis of the distal-end-part body to face the rotating shaft. The third aspect prescribes one preferred form of the stopper portion.

According to a fourth aspect of the invention, the stopper portion is a dent provided on the front end face and the stopped portion is a protrusion provided on the rear end face. The fourth aspect prescribes one preferred form of the stopper portion.

According to a fifth aspect of the invention, the stopper portion is a protrusion provided on the front end face and the stopped portion is a dent provided on the rear end face. The fifth aspect prescribes one preferred form of the stopper portion.

According to a sixth aspect of the invention, the contact member is attachably and detachably mounted on the distal-end-part body. The sixth aspect allows the contact member to be replaced.

According to a seventh aspect of the invention, the endoscope further comprises a cap that is mounted on the distal-end-part body, covers the elevator-housing space, and includes a wall portion defining an open window opened on a side thereof facing the treatment tool-guide surface, and the cap and the contact member are integrally molded. The seventh aspect prescribes that the cap and the contact member are formed by integral molding.

According to an eighth aspect of the invention, the cap is attachably and detachably mounted on the distal-end-part body. The eighth aspect allows the contact member to be replaced by the replacement of the cap.

According to a ninth aspect of the invention, the distal-end-part body is made of a metal material and the contact member is made of a resin material. The ninth aspect prescribes preferred materials of the distal-end-part body and the contact member.

According to a tenth aspect of the invention, the contact member includes a contact surface that includes a point of support in a case where the treatment tool led out of the treatment tool outlet is made to stand by the elevator, and a pressing surface that is capable of pressing and fixing the treatment tool in a state where the elevator is in the standing position. The tenth aspect prescribes a preferred form of the contact member.

According to an eleventh aspect of the invention, the treatment tool is pressed and fixed by the treatment tool-guide surface of the elevator and the pressing surface of the contact member. The eleventh aspect can press and fix the treatment tool by the treatment tool-guide surface and the pressing surface.

According to the endoscope of the aspect of the invention, it is possible to prevent the deformation of the contact member and to suppress a positional deviation between the contact member and the distal-end-part body.

### BRIEF DESCRIPTION OF THE DRAWINGS

Fig. 1 is a diagram showing the configuration of an endoscope system including an endoscope according to a first embodiment.
Fig. 2 is an enlarged perspective view of a distal end part.
Fig. 3 is an exploded perspective view showing the configuration of the distal end part shown in Fig. 2.
Fig. 4 is a perspective view of a distal-end-part body seen from an X(+) side.
Fig. 5 is an exploded perspective view of the distal-end-part body and an elevator.
Fig. 6 is a cross-sectional view of main portions showing a state where a treatment tool is made to stand by the elevator in the endoscope according to the first embodiment.
Fig. 7 is a cross-sectional view of main portions showing a state where a guide wire is fixed by the elevator in the endoscope according to the first embodiment.
Fig. 8 is a cross-sectional view of main portions showing a state where a treatment tool is made to stand by an elevator in an endoscope according to a second embodiment.
Fig. 9 is a cross-sectional view of main portions showing a state where a treatment tool is made to stand by an elevator in an endoscope according to a third embodiment.
Fig. 10 is a cross-sectional view of main portions showing a state where a treatment tool is made to stand by an elevator in an endoscope according to another embodiment.
Fig. 11 is an exploded perspective view showing the configuration of a distal end part of an endoscope according to a fourth embodiment.
Fig. 12 is an exploded perspective view showing the configuration of the distal end part of the endoscope according to the fourth embodiment.

### DESCRIPTION OF THE PREFERRED EMBODIMENTS

Endoscopes according to preferred embodiments of the invention will be described below with reference to the accompanying drawings.

### First embodiment

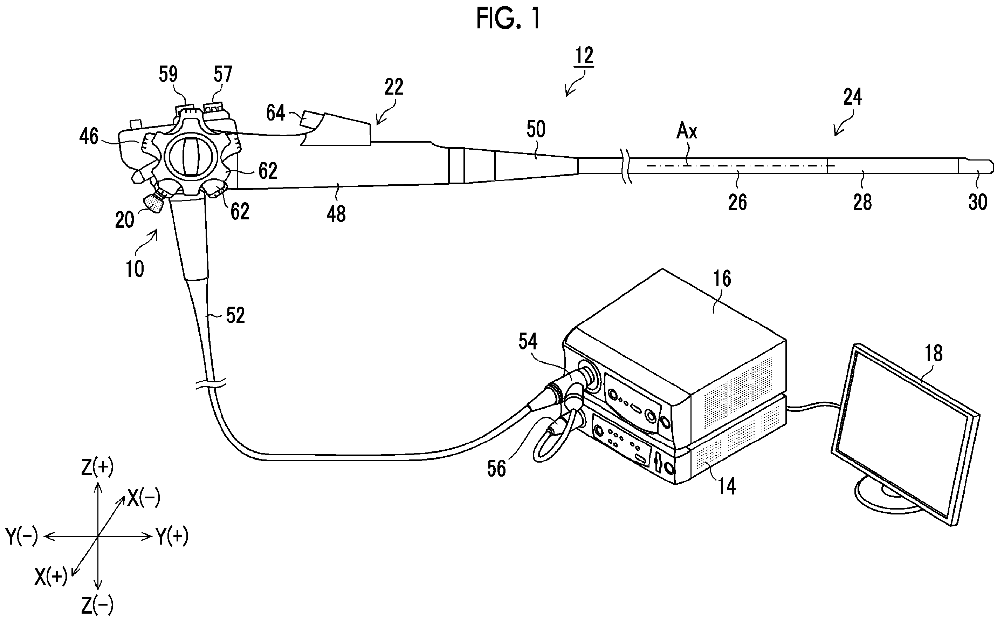

Fig. 1 is a diagram showing the configuration of an endoscope system 12 including an endoscope 10 according to an embodiment of the invention. The endoscope system 12 comprises an endoscope 10, a processor device 14, a light source device 16, and a display 18.

The endoscope 10 comprises an operation unit 22 that is provided with a standing operation lever 20 serving as an operation member and an insertion unit 24 that is provided on the distal end side of the operation unit 22 and is to be inserted into an object to be examined.

The insertion unit 24 has a longitudinal axis Ax extending toward a distal end from a proximal end, and comprises a soft part 26, a bendable part 28, and a distal end part 30 that are arranged in this order from the proximal end toward the distal end. The detailed configuration of the distal end part 30 will be described later, and the schematic configuration of the distal end part 30 will be described first.

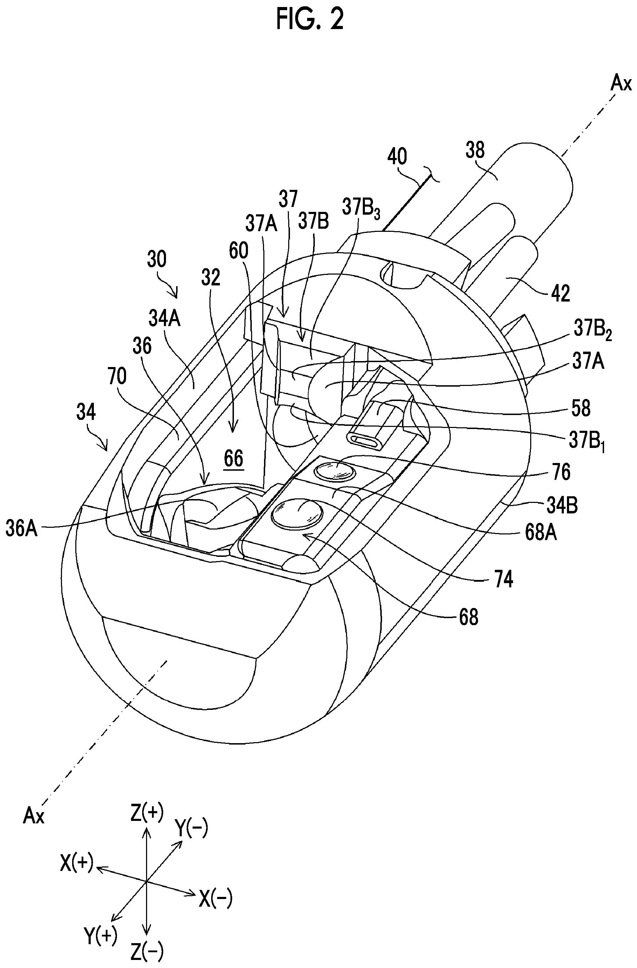

Fig. 2 is an enlarged perspective view of the distal end part 30. Here, the endoscope 10 (see Fig. 1) according to the embodiment is a side-viewing endoscope used as, for example, a duodenoscope, and the distal end part 30 shown in Fig. 2 includes the configuration of the side-viewing endoscope.

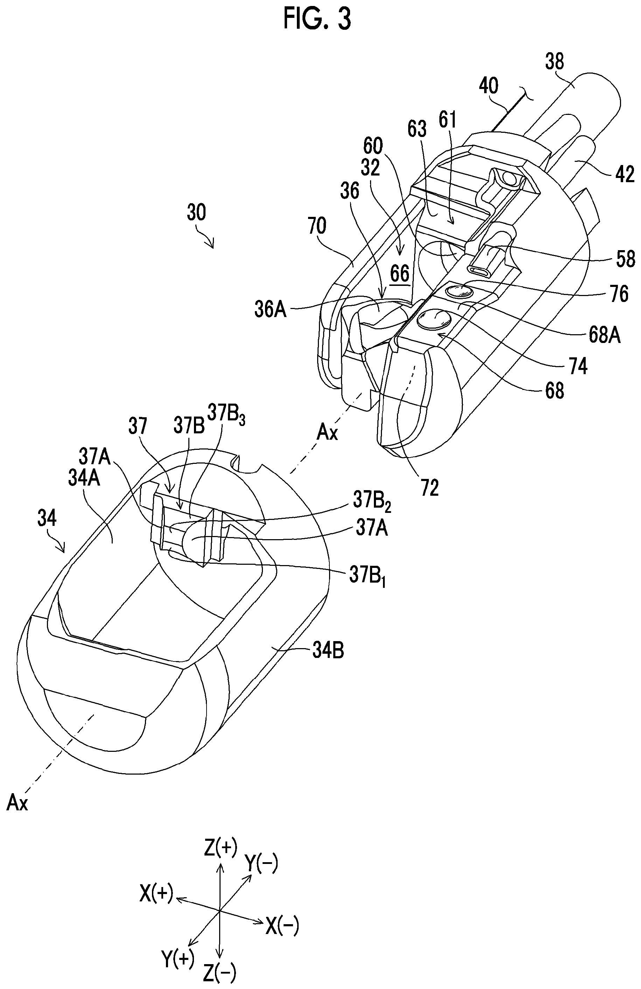

Further, Fig. 3 is an exploded perspective view showing the configuration of the distal end part 30 shown in Fig. 2. The distal end part 30 includes a distal-end-part body 32 and a cap 34 as shown in Fig. 3, and the cap 34 is mounted on the distal-end-part body 32, so that the distal end part 30 is formed. The distal-end-part body 32 is provided on the distal end side of the insertion unit 24 (see Fig. 1), and an elevator 36 including a treatment tool-guide surface 36A to be described later is provided in the distal-end-part body 32. Figs. 2 and 3 show a state where the elevator 36 is in a fallen position.

Further, Figs. 2 and 3 show various components provided in the insertion unit 24 of the endoscope 10 (see Fig. 1). Specifically, a treatment tool channel 38 that guides the distal end portion of a treatment tool (not shown) to the distal-end-part body 32, a standing operation wire 40 (hereinafter, referred to as a wire 40) that is used to perform an operation for changing the lead-out direction of the distal end portion of the treatment tool led out of the distal-end-part body 32, and an air/water supply tube 42 are provided. Furthermore, although not shown in Figs. 2 and 3, components, such as angle wires to be used to perform an operation for changing the bending direction of the bendable part 28 (see Fig. 1), a signal cable transmitting image signals, and a light guide transmitting illumination light, are also provided.

A three-dimensional Cartesian coordinate system including three axis directions (an X-axis direction, a Y-axis direction, and a Z-axis direction) will be used to make a description in this specification. For example, in Figs. 1 to 3, a Z(+) direction indicates an upward direction and a Z(-) direction indicates a downward direction. Further, an X(+) direction indicates a right direction and an X(-) direction indicates a left direction. Furthermore, a Y(+) direction indicates a direction toward the distal end of the distal end part 30 and a Y(-) direction indicates a direction toward the proximal end of the distal end part 30. A Y-axis direction including the Y(+) direction and the Y(-) direction is parallel to the direction of the longitudinal axis Ax of the insertion unit 24.

Returning to Fig. 1, the operation unit 22 has a substantially cylindrical shape as a whole. The operation unit 22 includes an operation unit body 46 on which the standing operation lever 20 is rotatably provided and a grip part 48 that is connected to the operation unit body 46, and the proximal end portion of the insertion unit 24 is provided on the distal end side of the grip part 48 through a bending-proof pipe 50. The grip part 48 is a part that is to be gripped by an operator during the operation of the endoscope 10.

Further, the operation unit body 46 is provided with a universal cable 52. A light source connector 54 is provided on the distal end side of the universal cable 52. The light source connector 54 is provided with an electrical connector 56 that is branched from the light source connector 54. The electrical connector 56 is connected to the processor device 14 and the light source connector 54 is connected to the light source device 16.

Furthermore, an air/water supply button 57 and a suction button 59 are provided on the operation unit body 46 side by side. In a case where the air/water supply button 57 is operated, air and water are supplied to the air/water supply tube 42 shown in Fig. 2 and air and water can be jetted from an air/water supply nozzle 58 provided in the distal-end-part body 32. The air/water supply button 57 shown in Fig. 1 is adapted to be operated in two stages, air can be supplied to the air/water supply tube 42 by one-stage operation of the air/water supply button 57, and water can be supplied to the air/water supply tube 42 by two-stage operation of the air/water supply button 57.

Further, in a case where the suction button 59 shown in Fig. 1 is operated, body fluid, such as blood, can be sucked through the treatment tool channel 38 from a suction port that is provided on a front end face 61 of the distal-end-part body 32 shown in Fig. 2 and also functions as a treatment tool outlet 60. The front end face 61 is the surface of the distal-end-part body 32 on which the treatment tool outlet 60 can be formed.

As shown in Fig. 1, a pair of angle knobs 62 and 62 used to bend the bendable part 28 is disposed on the operation unit body 46. The pair of angle knobs 62 and 62 is provided on the same shaft to be rotationally movable.

The standing operation lever 20 is rotatably provided on the same shaft as the angle knobs 62 and 62. The standing operation lever 20 is operated to rotate by the hand of an operator gripping the grip part 48. In a case where the standing operation lever 20 is operated to rotate, the wire 40 shown in Fig. 2 is operated to be pushed or pulled in conjunction with the rotating operation of the standing operation lever 20. The attitude of the elevator 36, which is connected to the distal end side of the wire 40, is changed between the fallen position shown in Fig. 2 and a standing position (not shown) by such an operation of the wire 40.

As shown in Fig. 1, the grip part 48 of the operation unit 22 is provided with a treatment tool inlet 64 into which the treatment tool is to be introduced. The treatment tool (not shown), which is introduced from the treatment tool inlet 64 so that the distal end portion of the treatment tool becomes a leading end, is inserted into the treatment tool channel 38 shown in Fig. 2 and is led out of the treatment tool outlet 60 provided on the distal-end-part body 32.

As shown in Fig. 1, the soft part 26 of the insertion unit 24 includes a spiral pipe (not shown) that is formed of a thin belt-like plate spirally wound and made of metal having elasticity. The outer portion of the spiral pipe is covered with a tubular mesh body woven with metal wires and the outer peripheral surface of the mesh body is covered with a covering made of a resin, so that the soft part 26 is formed.

The bendable part 28 of the insertion unit 24 includes a structure that is formed of a plurality of angle rings (not shown) connected to each other to be rotationally movable. The outer periphery of the structure is covered with a tubular mesh body woven with metal wires and the outer peripheral surface of the mesh body is covered with a tubular covering made of rubber, so that the bendable part 28 is formed. For example, four angle wires (not shown) are provided from the bendable part 28 formed as described above to the angle knobs 62 and 62 and these angle wires are pushed or pulled by the rotational moving operation of the angle knobs 62 and 62, so that the bendable part 28 is vertically and laterally bent.

The endoscope 10 according to the embodiment formed as described above is a side-viewing endoscope used as, for example, a duodenoscope, and the insertion unit 24 is inserted into an object to be examined through an oral cavity. The insertion unit 24 is inserted into the duodenum from the gullet through the stomach, so that treatment, such as predetermined examination or predetermined therapy, is performed.

Treatment tools, such as a pair of biopsy forceps including a cup provided at the distal end portion thereof and capable of being used to collect body tissue, a knife for endoscopic sphincterotomy (EST), and a contrast tube, can be exemplified as a treatment tool used for the endoscope 10 according to the embodiment.

Next, the structure of the distal end part 30 shown in Figs. 2 and 3 will be described in detail.

Although description is repeated, the distal end part 30 comprises the distal-end-part body 32 and the cap 34 that is attachably and detachably mounted on the distal-end-part body 32. The cap 34 comprises a wall portion 34B that is formed substantially in the shape of a tube of which the distal end side is sealed, and a substantially rectangular open window 34A is defined at a part of the outer peripheral surface thereof by the wall portion 34B. In a case where the cap 34 is mounted on the distal-end-part body 32 as shown in Fig. 2, the cap 34 covers an elevator-housing space 66 and the open window 34A is opened in the Z(+) direction that is a first direction orthogonal to the direction of the longitudinal axis Ax. Accordingly, the treatment tool outlet 60 of the distal-end-part body 32 communicates with the open window 34A through the elevator-housing space 66.

The cap 34 is made of an elastic material, for example, a rubber material, such as fluororubber or silicone rubber, or a resin material, such as polysulfone or polycarbonate. A protruding engaging portion (not shown) to be engaged with a groove-like engaged portion (not shown) formed on the distal-end-part body 32 is provided on the proximal end side of the cap 34 and the engaging portion is engaged with the engaged portion, so that the cap 34 is attachably and detachably mounted on the distal-end-part body 32.

The cap 34 comprises a contact member 37 molded integrally with the wall portion 34B. The contact member 37 is made of a resin material. The contact member 37 is disposed at the proximal end of the open window 34A on the side of the treatment tool outlet 60 corresponding to the Z(+) direction. The contact member 37 protrudes in the Y(+) direction as a whole. The contact member 37 is provided at a position facing the treatment tool-guide surface 36A in a case where the elevator 36 is in the standing position. "Molded integrally" means that a member is joined and a product (cap 34) is integrally molded without the use of adhesion or mechanical joining.

As shown in Fig. 2, the contact member 37 includes two side surfaces 37A and a protruding surface 37B positioned between the two side surfaces 37A. The two side surfaces 37A are positioned on the X(+) side and the X(-) side of the protruding surface 37B as seen in the Y(+) direction, and are inclined to spread in the X(+) direction and the X(-) direction toward the Y(-) side from the distal end of the protruding surface 37B.

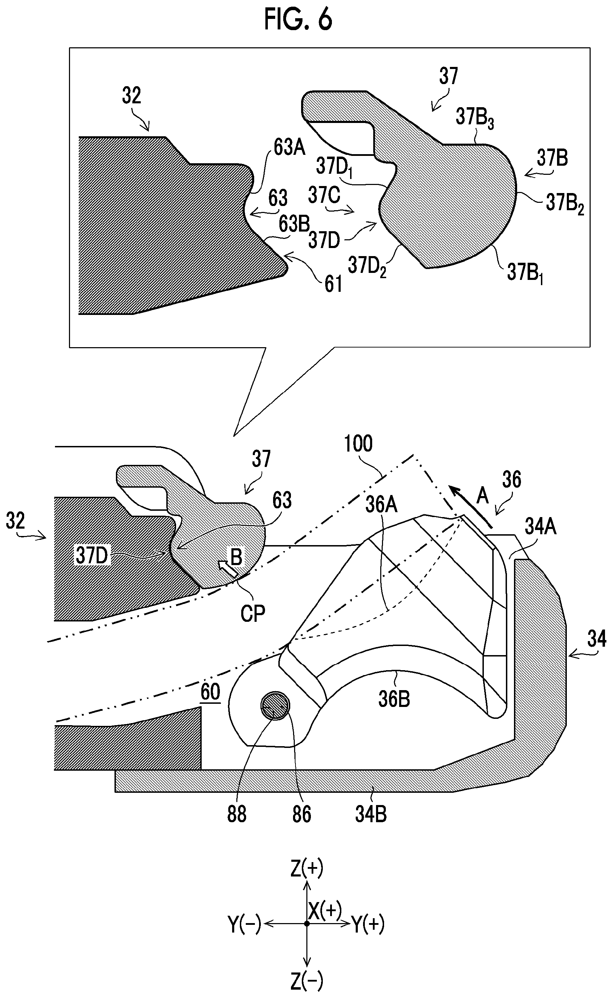

The protruding surface 37B includes a contact surface 37B₁, a pressing surface 37B₂, and an upper surface 37B₃ arranged in this order from the Z(-) side toward the Z(+) side. The contact surface 37B₁ is a surface that includes a point of support in a case where the treatment tool (not shown) led out of the treatment tool outlet 60 is made to stand by the elevator 36. The pressing surface 37B₂ is a surface that can press and fix the treatment tool in a state where the elevator 36 is in the standing position. The upper surface 37B₃ is a surface that is not in contact with the treatment tool (not shown) made to stand by the elevator 36. The pressing surface 37B₂ has a rounded quadrangular shape as seen in the Y(+) direction. The contact surface 37B₁ has a shape that is dented in a curved shape along the shape of the treatment tool outlet 60. However, the shape of the contact member 37 is not particularly limited. The contact member 37 comprises a rear end face 37C facing the front end face 61 of the distal-end-part body 32, and a stopped portion 37D is provided on the rear end face 37C (see Fig. 6).

After treatment using the endoscope 10 ends, the cap 34 is detached from the distal-end-part body 32, is washed and disinfected or sterilized or is discarded as a disposable.

The distal-end-part body 32 is made of a metal material having corrosion resistance. The distal-end-part body 32 includes a pair of partition walls 68 and 70 protruding toward the Y(+) side as shown in Fig. 3, and these partition walls 68 and 70 are disposed to face each other in the X-axis direction. Further, the elevator-housing space 66 housing the elevator 36 is provided between the partition walls 68 and 70. The elevator-housing space 66 is opened in the Z(+) direction that is the first direction orthogonal to the direction of the longitudinal axis Ax and the Z(-) direction.

A positional relationship between the partition wall 68 and the elevator-housing space 66 will be described. The partition wall 68 is disposed adjacent to the elevator-housing space 66 in the X-axis direction. Further, an illumination window 74 and an observation window 76 are provided on the upper surface 68A of the partition wall 68 corresponding to the Z(+) side so as to be adjacent to each other in the Y-axis direction. An area within the field of view in the Z(+) direction where the elevator-housing space 66 is opened can be observed through the observation window 76.

A positional relationship between the observation window 76 and the elevator-housing space 66 will be described. The observation window 76 and the elevator-housing space 66 are disposed adjacent to each other in the X-axis direction. The X-axis direction is a second direction orthogonal to the direction of the longitudinal axis Ax and orthogonal to the Z(+) direction that is the first direction. The above-mentioned air/water supply nozzle 58 is provided on the distal-end-part body 32 to face the observation window 76. Accordingly, the observation window 76 is washed with air and water jetted from the air/water supply nozzle 58.

An optical system-housing chamber 72 is provided in the partition wall 68. An illumination unit (not shown) and an image pickup unit (not shown) are housed in the optical system-housing chamber 72. The illumination unit comprises an illumination lens (not shown) provided on the side of the illumination window 74 close to the optical system-housing chamber 72, and a light guide (not shown) disposed so that a front end face of the light guide faces the illumination lens. The light guide is provided in the universal cable 52 from the insertion unit 24 of the endoscope 10 (see Fig. 1) through the operation unit 22, and the proximal end of the light guide is connected to the light source connector 54. Accordingly, in a case where the light source connector 54 is connected to the light source device 16, illumination light generated from the light source device 16 is transmitted to the illumination lens through the light guide and an area within the field of view present in the Z(+) direction is irradiated with the illumination light from the illumination window 74.

The above-mentioned image pickup unit comprises an image pickup optical system (not shown) that is provided in the observation window 76 and a complementary metal oxide semiconductor (CMOS) or charge coupled device (CCD) image pickup element (not shown). The distal end of the signal cable (not shown) is connected to the image pickup element, the signal cable is provided in the universal cable 52 from the insertion unit 24 of the endoscope 10 (see Fig. 1) through the operation unit 22, and the proximal end of the signal cable is connected to the electrical connector 56. Accordingly, in a case where the electrical connector 56 is connected to the processor device 14, the image pickup signals of a subject image obtained by the image pickup unit are transmitted to the processor device 14 through the signal cable. Then, the image pickup signals are displayed on the display 18 as a subject image after being subjected to image processing by the processor device 14.

A stopper portion 63 is provided on the front end face 61 of the distal-end-part body 32. The stopper portion 63 is engaged with the stopped portion (not shown) provided on the rear end face of the contact member 37.

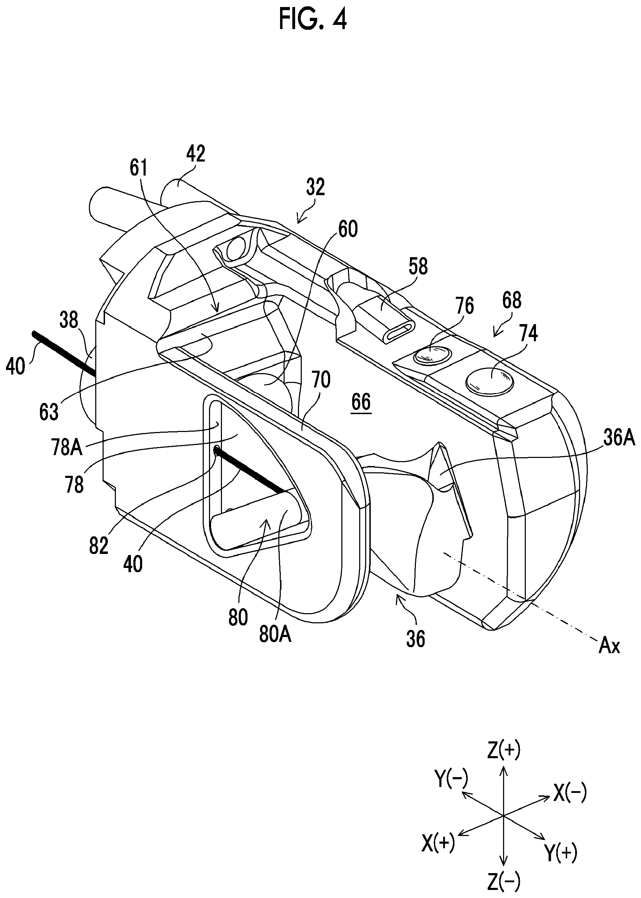

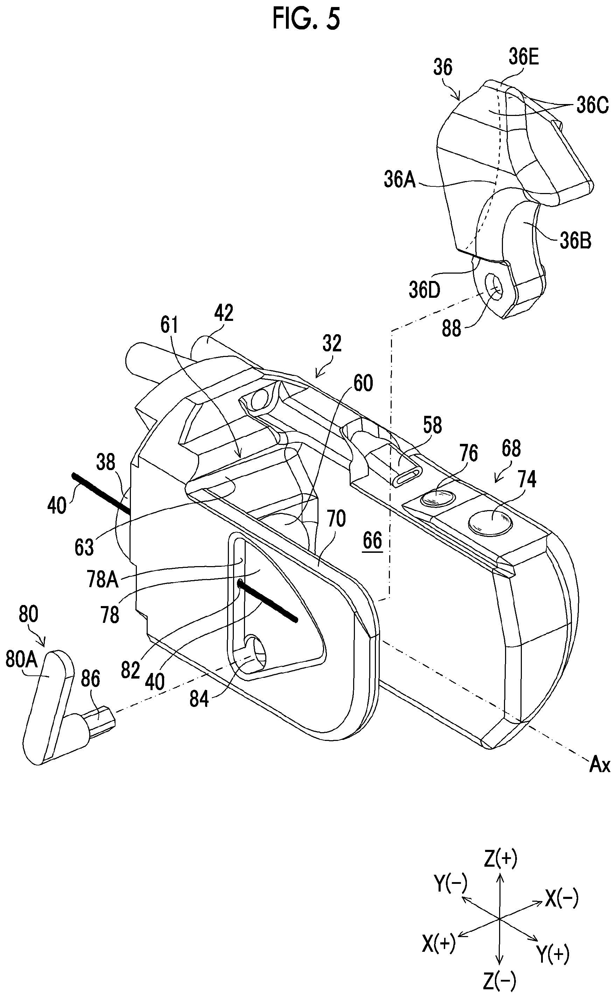

Next, the configuration of the elevator 36 will be described with reference to Figs. 4 and 5. Fig. 4 is a perspective view of the distal-end-part body 32 seen from the X(+) side, and Fig. 5 is an exploded perspective view of the distal-end-part body 32 and the elevator 36.

The elevator 36 is provided in the elevator-housing space 66 so as to be rotatable about a rotating shaft 86 (see Fig. 5), and is rotated between the standing position and the fallen position by the operation of the standing operation lever 20 (see Fig. 1). In a case where the elevator 36 is rotated toward the standing position, the treatment tool (not shown) led out of the treatment tool outlet 60 to the elevator-housing space 66 comes into contact with the treatment tool-guide surface 36A of the elevator 36, is guided in a direction toward the open window 34A of the cap 34 (see Fig. 2), and is led out of the open window 34A that is opened on the side facing the treatment tool-guide surface 36A. The treatment tool-guide surface 36A is formed on the upper surface (Z(+)side) of the elevator 36 in the shape of an arc curved upward toward the distal end side from the proximal end side, and is formed in a U shape in a cross section that is taken along a plane parallel to an X axis and a Z axis and is seen from the Y(+) side. The elevator 36 can easily guide the treatment tool due to the shape of the treatment tool-guide surface 36A.

A standing lever-housing chamber 78 is provided on the side surface of the distal-end-part body 32 corresponding to the X(+) side, and a standing lever 80 is housed in the standing lever-housing chamber 78. The standing lever-housing chamber 78 is covered with a protective plate (not shown) and is sealed.

The standing lever-housing chamber 78 has the shape of a fan-like recess, and a through-hole 82 where the wire 40 penetrates and is disposed is provided in the side surface 78A corresponding to the Y(-) side so as to extend in the Y-axis direction. The distal end of the wire 40 is inserted into the through-hole 82 and is fixed to a lever portion 80A of the standing lever 80.

Further, a through-hole 84, which penetrates the standing lever-housing chamber 78 and the elevator-housing space 66, is provided in the partition wall 70 so as to extend in the X-axis direction. The rotating shaft 86 of the standing lever 80 penetrates the through-hole 84 and is disposed in the through-hole 84, and is rotatably and pivotally supported. The distal end of the rotating shaft 86 is rotatably fitted to a hole 88 provided at the proximal end of the elevator 36. Accordingly, the standing lever 80 and the elevator 36 are connected to each other by the rotating shaft 86. Furthermore, in a case where the wire 40 is operated to be pushed or pulled by the standing operation lever 20 (see Fig. 1), the rotating shaft 86 is rotated together with the standing lever 80. Accordingly, the attitude of the elevator 36 is changed between the fallen position and the standing position (not shown). An O-ring (not shown) is disposed between the rotating shaft 86 and the through-hole 84, so that the entrance of gas and liquid into the elevator-housing space 66 and the standing lever-housing chamber 78 is prevented.

The elevator 36 comprises the treatment tool-guide surface 36A,an opposite surface 36B that is positioned on a side opposite to the side where the treatment tool-guide surface 36A is formed, two side surfaces 36C that are positioned on the X(+) side and the X(-) side and face each other, a proximal end face 36D that is positioned at the proximal end where the hole 88 is provided, and an upper surface 36E that is positioned on a side facing the proximal end face 36D.

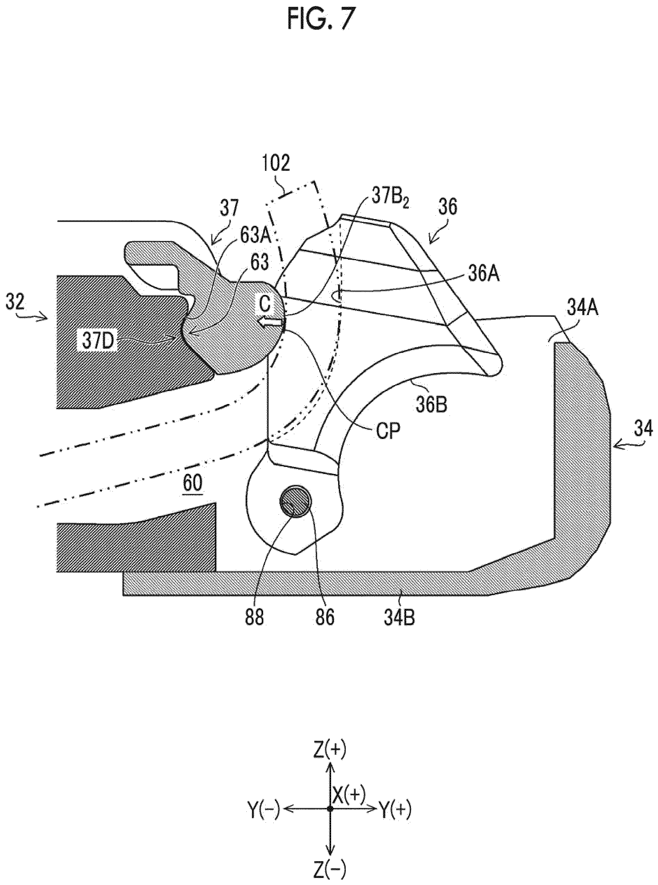

Next, each example of the endoscope 10 according to the embodiment that can prevent the deformation of the contact member and suppress a positional deviation will be described. Fig. 6 is a cross-sectional view of main portions showing a state where a treatment tool is made to stand by the elevator in the endoscope according to the first embodiment. Fig. 7 is a cross-sectional view of main portions showing a state where a guide wire is fixed by the elevator in the endoscope according to the first embodiment. Figs. 6 and 7 are cross-sectional views of main portions of the distal-end-part body 32, and are side views of the distal-end-part body 32 seen from the X(+) side.

As shown in Fig. 6, the contact member 37 is disposed on the front end face 61 of the distal-end-part body 32. The contact member 37 includes the rear end face 37C provided on the side opposite to the protruding surface 37B. The rear end face 37C is provided at a position facing the front end face 61 of the distal-end-part body 32. The stopped portion 37D is provided on the rear end face 37C. The stopper portion 63 to be engaged with the stopped portion 37D is provided on the front end face 61 of the distal-end-part body 32.

In a case where the elevator 36 is made to stand in the direction of an arrow A, a treatment tool 100 led out of the treatment tool outlet 60 is put into a standing state. The treatment tool 100 is in contact with a part of the contact surface 37B₁, and is put into a standing state while a contact point CP functions as the point of support. The contact member 37 receives a force applied in the direction of an arrow B from the contact point CP through the treatment tool 100 as the elevator 36 is operated. As shown by the arrow B, this force is the resultant force of a Y(-)-direction force component and a Z(+)-direction force component at the contact point CP. The magnitude of the Y(-)-direction force component and the magnitude of the Z(+)-direction force component are changed depending on the position of the elevator 36. As the elevator 36 is put into a standing state from a fallen state, the Y(-)-direction force component of the force received by the contact member 37 is increased and the Z(+)-direction force component thereof is reduced.

The Z(+)-direction force component causes the contact member 37 to be deformed and turned in the Z(+) direction and causes a positional deviation between the contact member 37 and the distal-end-part body 32. To prevent a positional deviation between the contact member 37 and the distal-end-part body 32, it is necessary to make the contact member 37 not be deformed and turned even in a case where the contact member 37 receives the Z(+)-direction force component. The deformation and turn of the contact member 37 is prevented by engagement between the stopped portion 37D and the stopper portion 63 in embodiments to be described below, so that the occurrence of a positional deviation is suppressed.

In the first embodiment, the stopped portion 37D has the shape of a protrusion tapered in the Y(-) direction in a cross-sectional view seen in the X-axis direction. The protrusion includes an inclined surface 37D₁ and an inclined surface 37D₂ that are inclined with respect to a Y axis. The inclined surface 37D₁ is formed of an inclined surface that faces in the Z(-) direction toward the Y(-) direction, and the inclined surface 37D₂ is formed of an inclined surface that faces in the Z(+) direction toward the Y(-) direction.

The stopper portion 63 has the shape of a dent gradually widened in the Y(+) direction, and is engaged with the stopped portion 37D. The stopper portion 63 includes an inclined surface 63A that faces the rotating shaft 86 toward the elevator 36, and an inclined surface 63B that faces the open window 34A toward the elevator 36. The inclined surface 63A is formed of an inclined surface that faces in the Z(+) direction toward the Y(+) direction. The inclined surface 63B is formed of an inclined surface that faces in the Z(-) direction toward the Y(+) direction.

The inclined surface 63A of the stopper portion 63 and the inclined surface 37D₁ of the stopped portion 37D are engaged with each other, and the inclined surface 63B of the stopper portion 63 and the inclined surface 37D₂ of the stopped portion 37D are engaged with each other.

In a case where the treatment tool 100 led out of the treatment tool outlet 60 is made to stand by the elevator 36, at least the inclined surface 63A of the stopper portion 63 receives the Z(+)-direction force component applied to the contact member 37. Since the inclined surface 63A resists the Z(+)-direction force component, the inclined surface 63A serves as a restriction surface that can restrict the movement of the contact member 37. The inclined surface 63A prevents the deformation of the contact member 37, and suppresses the occurrence of a positional deviation between the contact member 37 and the distal-end-part body 32.

Next, a case where a guide wire is used as a treatment tool will be described. A guide wire 102 is pressed and fixed by the treatment tool-guide surface 36A of the elevator 36 and the pressing surface 37B₂ of the contact member 37 in a case where the elevator 36 is made to stand as shown in Fig. 7. The Y(-)-direction force component of a force received by the contact member 37 is dominant. The contact member 37 is pressed against the distal-end-part body 32 by a force substantially parallel to the Y(-) direction. Since the Z(+)-direction force component is small and the inclined surface 63A receives the Z(+)-direction force component, the deformation of the contact member 37 is prevented. Accordingly, a positional deviation between the contact member 37 and the distal-end-part body 32 hardly occurs.

The angles of the inclined surfaces 63A and 63B of the stopper portion 63 with respect to the Y-axis direction and the angles of the inclined surfaces 37D₁ and 37D₂ of the stopped portion 37D with respect to the Y-axis direction can be appropriately changed without departing from the scope of the invention.

### Second embodiment

An endoscope according to a second embodiment will be described with reference to the drawing. The same components as those of the first embodiment will be denoted by the same reference numerals as those of the first embodiment, the detailed description thereof will be omitted, and a difference between the second embodiment and the first embodiment will be mainly described.

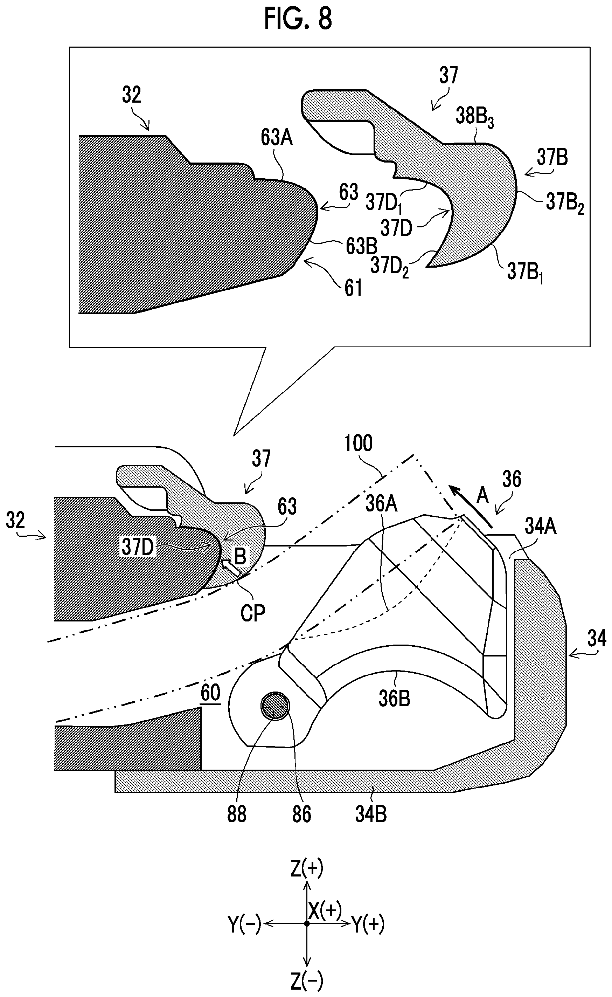

In the second embodiment, as shown in Fig. 8, a cap 34 comprises a contact member 37 molded integrally with a wall portion 34B as in the first embodiment. On the other hand, the shapes of a stopper portion 63 and a stopped portion 37D of the second embodiment are different from the shapes of the stopper portion 63 and the stopped portion 37D of the first embodiment.

In the second embodiment, the stopped portion 37D has the shape of a dent gradually widened in the Y(-) direction in a cross-sectional view seen in the X-axis direction. The dent includes an inclined surface 37D₁ and an inclined surface 37D₂ inclined with respect to the Y axis. The inclined surface 37D₁ is formed of an inclined surface that faces in the Z(+) direction toward the Y(-) direction, and the inclined surface 37D₂ is formed of an inclined surface that faces in the Z(-) direction toward the Y(-) direction.

On the other hand, the stopper portion 63 has the shape of a protrusion tapered in the Y(+) direction, and is engaged with the stopped portion 37D. The stopper portion 63 includes an inclined surface 63A that faces the open window 34A and the inclined surface 63B that faces the rotating shaft 86 toward the elevator 36. The inclined surface 63A is formed of an inclined surface that faces in the Z(-) direction toward the Y(+) direction, and the inclined surface 63B is formed of an inclined surface that faces in the Z(+) direction toward the Y(+) direction.

The inclined surface 63A of the stopper portion 63 and the inclined surface 37D₁ of the stopped portion 37D are engaged with each other and the inclined surface 63B of the stopper portion 63 and the inclined surface 37D₂ of the stopped portion 37D are engaged with each other.

In a case where a treatment tool 100 led out of the treatment tool outlet 60 is made to stand by the elevator 36, at least the inclined surface 63B of the stopper portion 63 receives a Z(+)-direction force component applied to the contact member 37. Since the inclined surface 63B resists the Z(+)-direction force component, the inclined surface 63B serves as a restriction surface that can restrict the movement of the contact member 37. The inclined surface 63B prevents the deformation of the contact member 37, and suppresses the occurrence of a positional deviation between the contact member 37 and the distal-end-part body 32.

A protruding surface 37B of the contact member 37 of the second embodiment has the same shape as the protruding surface 37B of the contact member 37 of the first embodiment. Even in the second embodiment, a guide wire 102 is pressed and fixed by the treatment tool-guide surface 36A of the elevator 36 and the pressing surface 37B₂ of the contact member 37 in a case where the elevator 36 is made to stand as in Fig. 7. Further, since the Z(+)-direction force component is small and the inclined surface 63B receives the Z(+)-direction force component, the deformation of the contact member 37 is prevented. Accordingly, a positional deviation between the contact member 37 and the distal-end-part body 32 hardly occurs.

The angles of the inclined surfaces 63A and 63B of the stopper portion 63 with respect to the Y-axis direction and the angles of the inclined surfaces 37D₁ and 37D₂ of the stopped portion 37D with respect to the Y-axis direction can be appropriately changed without departing from the scope of the invention.

### Third embodiment

An endoscope according to a third embodiment will be described with reference to the drawing. The same components as those of the first and second embodiments will be denoted by the same reference numerals as those of the first and second embodiments, the detailed description thereof will be omitted, and a difference between the third embodiment and the other embodiments will be mainly described.

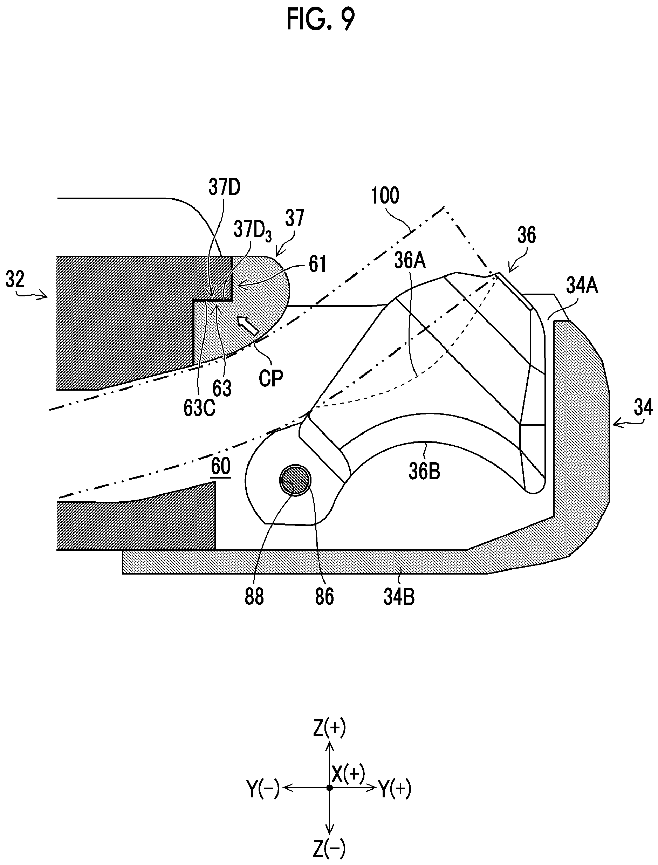

In the third embodiment, as shown in Fig. 9, a cap 34 comprises a contact member 37 molded integrally with a wall portion 34B as in the first embodiment. On the other hand, the shapes of a stopper portion 63 and a stopped portion 37D of the third embodiment are different from the shapes of the stopper portions 63 and the stopped portions 37D of the first and second embodiments.

In the third embodiment, the stopped portion 37D is formed of a parallel surface 37D₃ that extends toward the distal-end-part body 32 and is parallel to the longitudinal axis Ax of the distal-end-part body 32 (Y-axis direction) to face the open window 34A (Z(+) side) in a cross-sectional view seen in the X-axis direction.

In the third embodiment, the stopper portion 63 includes a restriction surface formed of a parallel surface 63C that extends toward the elevator 36 and is parallel to the longitudinal axis Ax of the distal-end-part body 32 (Y-axis direction) to face the rotating shaft 86 (Z(-) side) in a cross-sectional view seen in the X-axis direction.

The parallel surface 63C of the stopper portion 63 and the parallel surface 37D₃ of the stopped portion 37D are engaged with each other.

In a case where a treatment tool 100 led out of the treatment tool outlet 60 is made to stand by the elevator 36, at least the parallel surface 63C of the stopper portion 63 receives a Z(+)-direction force component applied to the contact member 37. Since the parallel surface 63C resists the Z(+)-direction force component, the parallel surface 63C serves as a restriction surface that can restrict the movement of the contact member 37. The parallel surface 63C prevents the deformation of the contact member 37, and suppresses the occurrence of a positional deviation between the contact member 37 and the distal-end-part body 32.

A protruding surface 37B of the contact member 37 of the third embodiment has the same shape as the protruding surface 37B of the contact member 37 of the first embodiment. Even in the third embodiment, a guide wire 102 is pressed and fixed by the treatment tool-guide surface 36A of the elevator 36 and the pressing surface 37B₂ of the contact member 37 in a case where the elevator 36 is made to stand as in Fig. 7. Further, since the Z(+)-direction force component is small and the parallel surface 63C receives the Z(+)-direction force component, the deformation of the contact member 37 is prevented. Accordingly, a positional deviation between the contact member 37 and the distal-end-part body 32 hardly occurs.

A case where the stopper portion 63 is formed of the parallel surface 63C parallel to the longitudinal axis Ax has been described, but the stopper portion 63 can be formed of an inclined surface that faces the rotating shaft 86 toward the elevator 36. This inclined surface (not shown) is formed of an inclined surface that faces in the Z(+) direction toward the Y(+) direction.

Next, an endoscope according to another embodiment will be described. The endoscope according to another embodiment includes a cap that comprises a contact member molded integrally with a wall portion as in the first to third embodiments. On the other hand, a part of the structure of the contact member is different from those of the first to third embodiments.

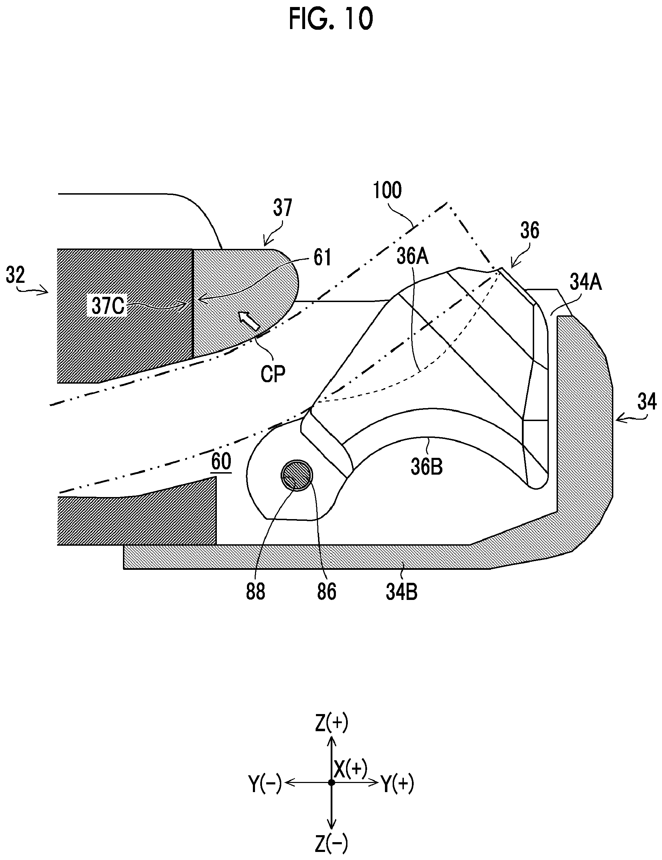

As shown in Fig. 10, a stopped portion is not provided on a rear end face 37C of a contact member 37. Further, a stopper portion is not provided on a front end face 61 of a distal-end-part body 32. In a case where joining strength, which is obtained in a case where the rear end face 37C of the contact member 37 is mounted on the front end face 61 of the distal-end-part body 32, has magnitude resisting a Z(+)-direction force component that is caused by an operation for making the elevator 36 stand, the deformation of the contact member 37 is prevented. Accordingly, a positional deviation between the contact member 37 and the distal-end-part body 32 can be prevented. For example, chemical joining using an adhesive or the like, mechanical joining, such as screwing, and a combination thereof can be applied to the mounting of the contact member 37 on the distal-end-part body 32.

In a case where the cap comprises the contact member molded integrally with the wall portion in the first to third embodiments and another embodiment, it is advantageous in terms of the following.

In a case where the cap 34 includes the contact member 37 and, for example, the cap 34 is detached from the distal-end-part body 32, a portion corresponding to the contact member 37 becomes an open space. Since a washing brush easily has access to the elevator-housing space 66, the washability of the distal-end-part body 32 is improved.

In a case where the contact member 37 includes a contact surface 37B₁ to be in contact with a treatment tool 100 and, particularly, a metal part is in contact with the contact surface 37B₁, the contact surface 37B₁ is likely to be subjected to damage, such as a scratch. Even though the contact surface 37B₁ is subjected to damage, the contact member 37 can be renewed by the replacement of the cap 34 with a new cap 34.

### Fourth embodiment

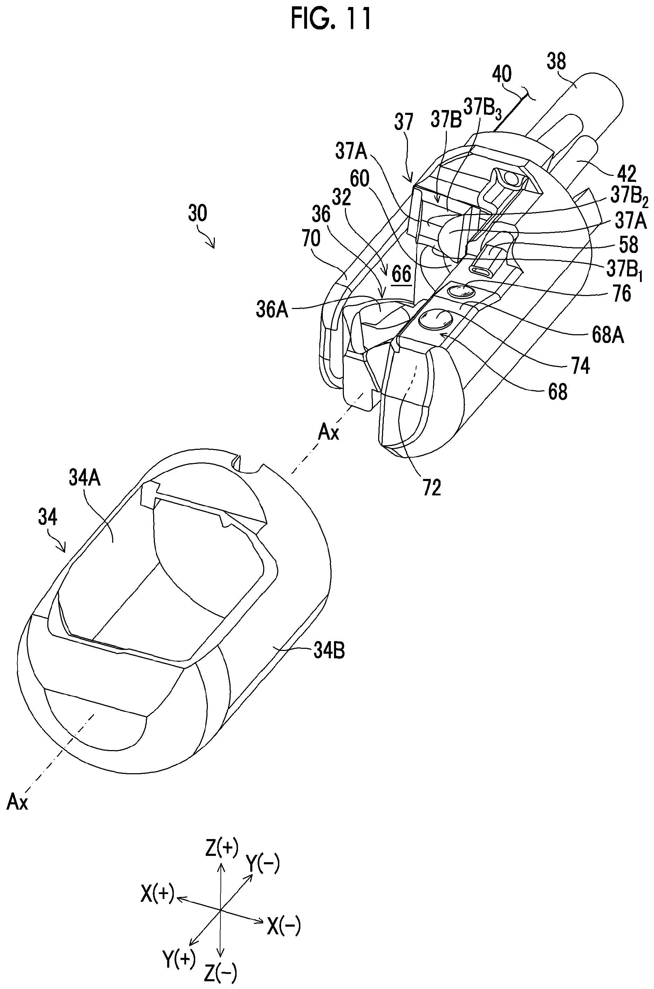

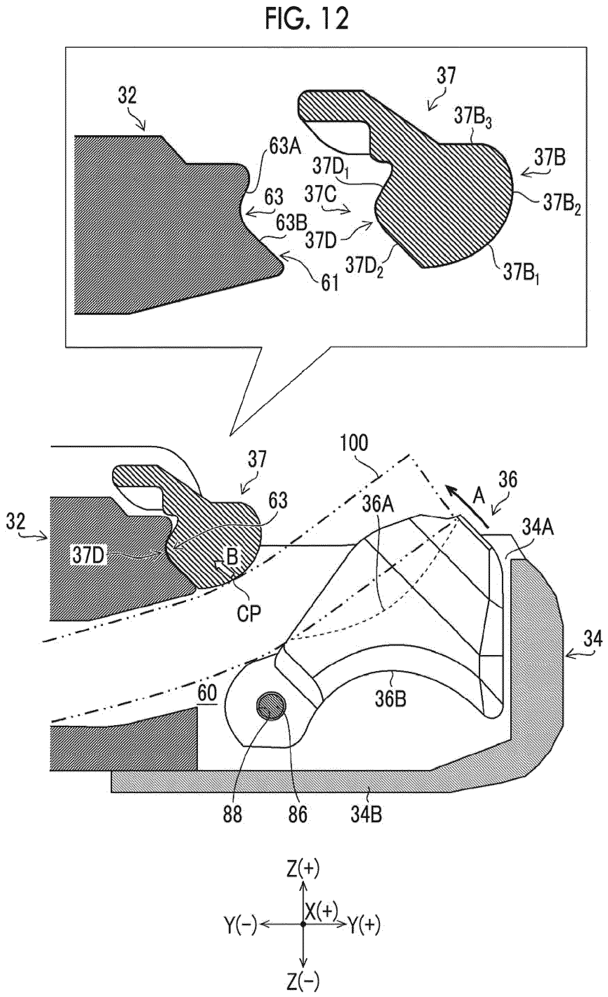

An endoscope according to a fourth embodiment will be described with reference to Figs. 11 and 12. The same components as those of the first to third embodiments will be denoted by the same reference numerals as those of the first to third embodiments, the detailed description thereof will be omitted, and a difference between the fourth embodiment and the other embodiments will be mainly described.

In the fourth embodiment, as shown in Fig. 11, a contact member 37 is provided on a front end face 61 (see Fig. 12) of a distal-end-part body 32 unlike in the first to third embodiments. The contact member 37 is disposed on the front end face 61 on the side of the treatment tool outlet 60 corresponding to the Z(+) direction. The contact member 37 protrudes in the Y(+) direction as a whole. The contact member 37 is provided at a position facing the treatment tool-guide surface 36A in a case where the elevator 36 is in the standing position.

As shown in Fig. 11, the contact member 37 includes two side surfaces 37A and a protruding surface 37B positioned between the two side surfaces 37A.

The protruding surface 37B includes a contact surface 37B₁, a pressing surface 37B₂, and an upper surface 37B₃ arranged in this order from the Z(-) side toward the Z(+) side. The contact surface 37B₁ is a surface that includes a contact point CP (see Fig. 12) serving as a point of support in a case where a treatment tool (not shown) led out of the treatment tool outlet 60 is made to stand by the elevator 36.

The pressing surface 37B₂ is a surface that can press and fix the treatment tool in a state where the elevator 36 is in the standing position. The upper surface 37B₃ is a surface that is not in contact with the treatment tool (not shown) made to stand by the elevator 36. The pressing surface 37B₂ has a rounded quadrangular shape as seen in the Y(+) direction. The contact surface 37B₁ has a shape that is dented in a curved shape along the shape of the treatment tool outlet 60. However, the shape of the contact member 37 is not particularly limited.

The contact member 37 comprises a rear end face 37C (see Fig. 12) facing the front end face 61 of the distal-end-part body 32. A stopped portion 37D is provided on the rear end face 37C of the contact member 37.

As shown in Fig. 12, the contact member 37 is formed of a member separate from the cap 34 and the distal-end-part body 32. Further, the contact member 37 can be made of a resin material like the cap 34, or can be made of an inexpensive metal material for metal injection molding (MIM) or the like. Alternatively, quenched steel or the like can be used for the contact member 37, or the contact member 37 can be made of a scratch-resistant material.

The contact member 37 is disposed on the front end face 61 of the distal-end-part body 32. The contact member 37 includes the rear end face 37C provided on the side opposite to the protruding surface 37B. The rear end face 37C is provided at a position facing the front end face 61 of the distal-end-part body 32. The stopped portion 37D is provided on the rear end face 37C. The stopper portion 63 to be engaged with the stopped portion 37D is provided on the front end face 61 of the distal-end-part body 32.

In the fourth embodiment, as in the first embodiment, the stopped portion 37D of the contact member 37 has the shape of a protrusion tapered in the Y(-) direction in a cross-sectional view seen in the X-axis direction. The protrusion includes an inclined surface 37D₁ and an inclined surface 37D₂ that are inclined with respect to the Y axis. The inclined surface 37D₁ is formed of an inclined surface that faces in the Z(-) direction toward the Y(-) direction, and the inclined surface 37D₂ is formed of an inclined surface that faces in the Z(+) direction toward the Y(-) direction.

The stopper portion 63 of the front end face 61 has the shape of a dent gradually widened in the Y(+) direction, and is engaged with the stopped portion 37D. The stopper portion 63 includes an inclined surface 63A that faces the rotating shaft 86 toward the elevator 36, and an inclined surface 63B that faces the open window 34A toward the elevator 36. The inclined surface 63A is formed of an inclined surface that faces in the Z(+) direction toward the Y(+) direction. The inclined surface 63B is formed of an inclined surface that faces in the Z(-) direction toward the Y(+) direction.

The inclined surface 63A of the stopper portion 63 and the inclined surface 37D₁ of the stopped portion 37D are engaged with each other and the inclined surface 63B of the stopper portion 63 and the inclined surface 37D₂ of the stopped portion 37D are engaged with each other.

In a case where a treatment tool 100 led out of the treatment tool outlet 60 is made to stand by the elevator 36, at least the inclined surface 63A of the stopper portion 63 receives a Z(+)-direction force component applied to the contact member 37. Since the inclined surface 63A resists the Z(+)-direction force component, the inclined surface 63A serves as a restriction surface that can restrict the movement of the contact member 37. The inclined surface 63A prevents the deformation of the contact member 37, and suppresses the occurrence of a positional deviation between the contact member 37 and the distal-end-part body 32.

For example, chemical joining using an adhesive or the like, mechanical joining, such as screwing, and a combination thereof can be applied to the mounting of the contact member 37 on the distal-end-part body 32. It is preferable that the contact member 37 is attachably and detachably mounted on the distal-end-part body 32. In a case where the contact surface 37B₁ or the like of the contact member 37 is subjected to damage or the like, the contact member 37 can be replaced with a new contact member 37.

In a case where the contact member 37 is mounted on the distal-end-part body 32 by mechanical joining, such as screwing, the attachable and detachable mounting can be easily realized. In the case of screwing, for example, a screw penetrates the contact member 37 from the elevator-housing space 66, reaches the distal-end-part body 32, and mounts the contact member 37 on the distal-end-part body 32. It is preferable that the head portion of a screw is embedded in the contact member 37. The interference between the screw and the treatment tool can be prevented.

The stopped portion 37D and the stopper portion 63, which are the same as those of the first embodiment, have been described in the fourth embodiment. However, the stopped portion 37D and the stopper portion 63 are not limited to the shapes thereof, and the stopped portion 37D and the stopper portion 63, which are the same as those of the second and third embodiments, can be applied.

The invention has been described above, but the invention is not limited to the above-mentioned embodiments. It goes without saying that the invention may have various improvements and modifications without departing from the scope of the invention.

### Explanation of References

10: endoscope
12: endoscope system
14: processor device
16: light source device
18: display
20: standing operation lever
22: operation unit
24: insertion unit
26: soft part
28: bendable part
30: distal end part
32: distal-end-part body
34: cap
34A: open window
34B: wall portion
36: elevator
36A: treatment tool-guide surface
36B: opposite surface
36C: side surface
36D: proximal end face
36E: upper surface
37: contact member
37A: side surface
37B: protruding surface
37B₁: contact surface
37B₂: pressing surface
37B₃: upper surface
37C: rear end face
37D: stopped portion
37D₁: inclined surface
37D₂: inclined surface
37D₃: parallel surface
38: treatment tool channel
40: wire
42: air/water supply tube
46: operation unit body
48: grip part
50: bending-proof pipe
52: universal cable
54: light source connector
56: electrical connector
57: air/water supply button
58: air/water supply nozzle
59: suction button
60: treatment tool outlet
61: front end face
62: angle knob
63: stopper portion
63A: inclined surface
63B: inclined surface
63C: parallel surface
64: treatment tool inlet
66: elevator-housing space
68: partition wall
68A: upper surface
70: partition wall
72: optical system-housing chamber
74: illumination window
76: observation window
78: standing lever-housing chamber
78A: side surface
80: standing lever
80A: lever portion
82: through-hole
84: through-hole
86: rotating shaft
88: hole
100: treatment tool
102: guide wire
Ax: longitudinal axis
A: arrow
B: arrow
CP: contact point

## Claims

1. An endoscope comprising:
an operation unit (22) that is provided with an operation member;
an insertion unit (24) that is provided on a distal end side of the operation unit (22) and is to be inserted into an object to be examined;
a distal-end-part body (32) which is positioned at a distal end of the insertion unit (24) and where a front end face (61) on which at least a treatment tool outlet (60) is formed and an elevator-housing space (66) communicating with the treatment tool outlet (60) are formed;
an elevator (36) that is disposed in the elevator-housing space (66), is rotatable about a rotating shaft (86) between a fallen position and a standing position, and includes a treatment tool-guide surface (36A); and
a contact member (37) that is disposed on the front end face (61) of the distal-end-part body (32) and is provided at a position facing the treatment tool-guide surface (36A) in a case where the elevator (36) is in the standing position,
wherein the contact member (37) includes a stopped portion (37D) that is provided on a rear end face (37C) thereof facing the front end face (61) of the distal-end-part body (32),
the distal-end-part body (32) includes a stopper portion (63) that is provided on the front end face (61),
the stopper portion (63) including a restriction surface and configured to be engaged with the stopped portion (37D), and
the restriction surface restricts at least movement of the contact member (37) in an upward direction in which the elevator (36) is made to stand with a force received from a treatment tool (100) in a case where the treatment tool (100) which is led out of the treatment tool outlet (60) is made to stand by the elevator (36), and
a cap (34) that is mounted on the distal-end-part body (32) covers the elevator-housing space (66), and includes a wall portion (34B) defining an open window (34A) opened on a side thereof facing the treatment tool-guide surface (36A), wherein the cap (34) and the contact member (37) are integrally molded.

2. The endoscope according to claim 1,
wherein the restriction surface is formed of an inclined surface (63A, 63B) that faces the rotating shaft (86) toward the elevator (36).

3. The endoscope according to claim 1,
wherein the restriction surface is formed of a parallel surface (63C) that extends toward the elevator (36) and is parallel to a direction of a longitudinal axis (Ax) of the distal-end-part body (32) to face the rotating shaft (86).

4. The endoscope according to claim 2,
wherein the stopper portion (63) is a dent provided on the front end face (61) and the stopped portion (37D) is a protrusion provided on the rear end face (37C).

5. The endoscope according to claim 2,
wherein the stopper portion (63) is a protrusion provided on the front end face (61) and the stopped portion (37D) is a dent provided on the rear end face (37C).

6. The endoscope according to any one of claims 1 to 5,
wherein the contact member (37) is attachably and detachably mounted on the distal-end-part body (32).

7. The endoscope according to claim 1,
wherein the cap (34) is attachably and detachably mounted on the distal-end-part body (32).

8. The endoscope according to any one of claims 1 to 7,
wherein the distal-end-part body (32) is made of a metal material and the contact member (37) is made of a resin material.

9. The endoscope according to any one of claims 1 to 8,
wherein the contact member (37) includes a contact surface (37B1) that includes a point of support in a case where the treatment tool (100) led out of the treatment tool outlet (60) is made to stand by the elevator (36), and a pressing surface (37B2) that is capable of pressing and fixing the treatment tool (100) in a state where the elevator (36) is in the standing position.

10. The endoscope according to claim 9,
wherein the treatment tool (100) is pressed and fixed by the treatment tool-guide surface (36A) of the elevator (36) and the pressing surface (37B2) of the contact member (37).

## Patentansprüche

1. Endoskop, umfassend:
eine Betätigungseinheit (22), die mit einem Betätigungselement versehen ist;
eine Einführeinheit (24), die auf einer distalen Endseite der Betätigungseinheit (22) vorgesehen ist und in ein zu untersuchendes Objekt eingeführt werden soll;
einen distalen Endteilkörper (32), der an einem distalen Ende der Einführeinheit (24) positioniert ist und an dem eine vordere Endfläche (61), an der mindestens ein Behandlungswerkzeugauslass (60) gebildet ist, und ein Hebergehäuseraum (66), der mit dem Behandlungswerkzeugauslass (60) kommuniziert, gebildet sind;
einen Heber (36), der in dem Hebergehäuseraum (66) angeordnet ist, um eine Drehwelle (86) zwischen einer gesenkten Position und einer stehenden Position drehbar ist und eine Behandlungswerkzeug-Führungsfläche (36A) enthält; und
ein Kontaktelement (37), das an der vorderen Endfläche (61) des distalen Endteilkörpers (32) angeordnet ist und in einem Fall, in dem der Heber (36) in der stehenden Position ist, an einer Position, die der Behandlungswerkzeug-Führungsfläche (36A) zugewandt ist, vorgesehen ist,
wobei das Kontaktelement (37) einen gestoppten Abschnitt (37D), der an einer hinteren Endfläche (37C) davon, die der vorderen Endfläche (61) des distalen Endteilkörpers (32) zugewandt ist, vorgesehen ist, enthält,
der distale Endteilkörper (32) einen Stopperabschnitt (63), der an der vorderen Endfläche (61) vorgesehen ist, enthält,
der Stopperabschnitt (63) eine Begrenzungsfläche enthält und so konfiguriert ist, dass er mit dem gestoppten Abschnitt (37D) in Eingriff steht, und
die Begrenzungsfläche mindestens Bewegung des Kontaktelements (37) in einer Aufwärtsrichtung, in der der Heber (36) mit einer von einem Behandlungswerkzeug (100) empfangenen Kraft zum Stehen gebracht wird, in einem Fall, in dem das Behandlungswerkzeug (100), das aus dem Behandlungswerkzeugauslass (60) herausgeführt wird, durch den Heber (36) zum Stehen gebracht wird, begrenzt, und
eine Kappe (34), die an dem distalen Endteilkörper (32) montiert ist, den Hebergehäuseraum (66) abdeckt und einen Wandabschnitt (34B), der ein offenes Fenster (34A), das auf einer Seite davon, die der Behandlungswerkzeug-Führungsfläche (36A) zugewandt ist, geöffnet ist, definiert, enthält, wobei die Kappe (34) und das Kontaktelement (37) integral geformt sind.

2. Endoskop nach Anspruch 1,
wobei die Begrenzungsfläche aus einer geneigten Oberfläche (63A, 63B), die der Drehwelle (86) zu dem Heber (36) hin zugewandt ist, gebildet ist.

3. Endoskop nach Anspruch 1,
wobei die Begrenzungsfläche aus einer parallelen Oberfläche (63C), die sich zu dem Heber (36) hin erstreckt und parallel zu einer Richtung einer Längsachse (Ax) des distalen Endteilkörpers (32) ist, gebildet ist, um der Drehwelle (86) zugewandt zu sein.

4. Endoskop nach Anspruch 2,
wobei der Stopperabschnitt (63) eine Vertiefung, die an der vorderen Endfläche (61) vorgesehen ist, ist, und der gestoppte Abschnitt (37D) ein Vorsprung, der an der hinteren Endfläche (37C) vorgesehen ist, ist.

5. Endoskop nach Anspruch 2,
wobei der Stopperabschnitt (63) ein Vorsprung, der an der vorderen Endfläche (61) vorgesehen ist, ist, und der gestoppte Abschnitt (37D) eine Vertiefung, die an der hinteren Endfläche (37C) vorgesehen ist, ist.

6. Endoskop nach einem der Ansprüche 1 bis 5,
wobei das Kontaktelement (37) anbringbar und abnehmbar an dem distalen Endteilkörper (32) montiert ist.

7. Endoskop nach Anspruch 1,
wobei die Kappe (34) anbringbar und abnehmbar an dem distalen Endteilkörper (32) montiert ist.

8. Endoskop nach einem der Ansprüche 1 bis 7,
wobei der distale Endteilkörper (32) aus einem Metallmaterial hergestellt ist und das Kontaktelement (37) aus einem Harzmaterial hergestellt ist.

9. Endoskop nach einem der Ansprüche 1 bis 8,
wobei das Kontaktelement (37) eine Kontaktfläche (37B1), die einen Stützpunkt in einem Fall, in dem das Behandlungswerkzeug (100), das aus dem Behandlungswerkzeugauslass (60) herausgeführt wurde, durch den Heber (36) zum Stehen gebracht wird, enthält, und eine Druckfläche (37B2), die in der Lage ist, das Behandlungswerkzeug (100) in einem Zustand, in dem der Heber (36) in der stehenden Position ist, zu drücken und zu fixieren, enthält.

10. Endoskop nach Anspruch 9,
wobei das Behandlungswerkzeug (100) durch die Behandlungswerkzeug-Führungsfläche (36A) des Hebers (36) und die Druckfläche (37B2) des Kontaktelements (37) gedrückt und fixiert wird.

## Revendications

1. Endoscope comprenant :
une unité d'opération (22) qui est pourvue d'un élément d'opération ;
une unité d'insertion (24) qui est prévue sur un côté d'extrémité distale de l'unité d'opération (22) et doit être insérée dans un objet à examiner ;
un corps de partie d'extrémité distale (32) qui est positionné à une extrémité distale de l'unité d'insertion (24) et où une face d'extrémité avant (61) sur laquelle au moins une sortie d'outil de traitement (60) est formée et un espace de logement d'élévateur (66) communiquant avec la sortie d'outil de traitement (60) sont formés ;
un élévateur (36) qui est disposé dans l'espace de logement d'élévateur (66), est rotatif autour d'un arbre rotatif (86) entre une position couchée et une position debout, et inclut une surface de guidage d'outil de traitement (36A) ; et
un élément de contact (37) qui est disposé sur la face d'extrémité avant (61) du corps de partie d'extrémité distale (32) et est prévu à une position faisant face à la surface de guidage d'outil de traitement (36A) dans un cas où l'élévateur (36) est dans la position debout,
dans lequel l'élément de contact (37) inclut une partie arrêtée (37D) qui est prévue sur une face d'extrémité arrière (37C) de celui-ci faisant face à la face d'extrémité avant (61) du corps de partie d'extrémité distale (32),
le corps de partie d'extrémité distale (32) inclut une partie de butée (63) qui est prévue sur la face d'extrémité avant (61),
la partie de butée (63) incluant une surface de retenue et configurée pour être engagée avec la partie arrêtée (37D), et
la surface de retenue limite au moins le mouvement de l'élément de contact (37) dans une direction vers le haut dans laquelle l'élévateur (36) est immobilisé avec une force reçue d'un outil de traitement (100) dans un cas où l'outil de traitement (100) qui est sorti de la sortie d'outil de traitement (60) est mis en position debout par l'élévateur (36), et
un capuchon (34) qui est monté sur le corps de partie d'extrémité distale (32) couvre l'espace de logement d'élévateur (66), et inclut une partie de paroi (34B) définissant une fenêtre ouverte (34A) ouverte sur un côté de celui-ci faisant face à la surface de guidage d'outil de traitement (36A), où le capuchon (34) et l'élément de contact (37) sont moulés intégralement.

2. Endoscope selon la revendication 1,
dans lequel la surface de retenue est formée d'une surface inclinée (63A, 63B) qui fait face à l'arbre rotatif (86) vers l'élévateur (36).

3. Endoscope selon la revendication 1,
dans lequel la surface de retenue est formée d'une surface parallèle (63C) qui s'étend vers l'élévateur (36) et est parallèle à une direction d'un axe longitudinal (Ax) du corps de partie d'extrémité distale (32) pour faire face à l'arbre rotatif (86).

4. Endoscope selon la revendication 2,
dans lequel la partie de butée (63) est un creux prévu sur la face d'extrémité avant (61) et la partie arrêtée (37D) est une saillie prévue sur la face d'extrémité arrière (37C).

5. Endoscope selon la revendication 2,
dans lequel la partie de butée (63) est une saillie prévue sur la face d'extrémité avant (61) et la partie arrêtée (37D) est un creux prévu sur la face d'extrémité arrière (37C).

6. Endoscope selon l'une quelconque des revendications 1 à 5,
dans lequel l'élément de contact (37) est monté de manière attachable et détachable sur le corps de partie d'extrémité distale (32).

7. Endoscope selon la revendication 1,
dans lequel le capuchon (34) est monté de manière attachable et détachable sur le corps de partie d'extrémité distale (32).

8. Endoscope selon l'une quelconque des revendications 1 à 7,
dans lequel le corps de partie d'extrémité distale (32) est constitué d'un matériau métallique et l'élément de contact (37) est constitué d'un matériau de résine.

9. Endoscope selon l'une quelconque des revendications 1 à 8,
dans lequel l'élément de contact (37) inclut une surface de contact (37B1) qui inclut un point d'appui dans un cas où l'outil de traitement (100) sorti de la sortie d'outil de traitement (60) est mis en position debout par l'élévateur (36), et une surface de pression (37B2) qui est capable de presser et de fixer l'outil de traitement (100) dans un état où l'élévateur (36) est dans la position debout.

10. Endoscope selon la revendication 9,
dans lequel l'outil de traitement (100) est pressé et fixé par la surface de guidage d'outil de traitement (36A) de l'élévateur (36) et la surface de pression (37B2) de l'élément de contact (37).
